(19) Europäisches Patentamt
European Patent Office
Office européen des brevets

(11) Veröffentlichungsnummer: **0 276 700**
**A1**

# (12) EUROPÄISCHE PATENTANMELDUNG

(21) Anmeldenummer: **88100503.7**

(51) Int. Cl.4: **C07D 215/56**, A61K 31/47

(22) Anmeldetag: **15.01.88**

(30) Priorität: **28.01.87 DE 3702393**

(43) Veröffentlichungstag der Anmeldung:
**03.08.88 Patentblatt 88/31**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Schriewer, Michael, Dr.**
**Am Thelen Siefen 1a**
**D-5068 Odenthal(DE)**
Erfinder: **Grohe, Klaus, Dr.**
**Am Wasserturm 10**
**D-5058 Odenthal(DE)**
Erfinder: **Petersen, Uwe, Dr.**
**Auf dem Forst 4**
**D-5090 Leverkusen 1(DE)**
Erfinder: **Haller, Ingo, Dr.**
**Dornröschenweg 4**
**D-5600 Wuppertal 1(DE)**
Erfinder: **Metzger, Karl Georg, Dr.**
**Pahlkestrasse 75**
**D-5600 Wuppertal 1(DE)**
Erfinder: **Endermann, Rainer, Dr.**
**In den Birken 152a**
**D-5600 Wuppertal 1(DE)**
Erfinder: **Zeiler, Hans-Joachim, Dr.**
**Elsbeeker Strasse 46**
**D-5620 Belbert 15(DE)**

(54) **8-Cyano-1-cyclopropyl-1,4-dihydro-4-oxo-3-chinolincarbonsäuren, Verfahren zu ihrer Herstellung und diese enthaltende antibakterielle Mittel.**

(57) Die Erfindung betrifft neue 8-Cyano-1-cyclopropyl-1,4-dihydro-4-oxo-3-chinolincarbonsäurederivate der Formel I

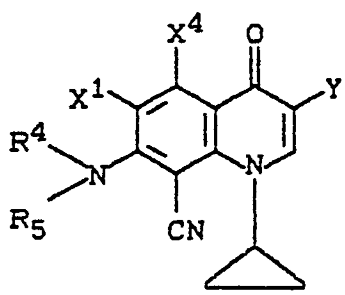

(I)

in welcher Y, $X^1$, $X^4$, $R^4$ und $R^5$ die in der Beschreibung angegebene Bedeutung haben, Verfahren zu ihrer

EP 0 276 700 A1

Herstellung sowie diese enthaltende antibakterielle Mittel.

# 8-Cyano-1-cyclopropyl-1,4-dihydro-4-oxo-3-chinolincarbonsäuren, Verfahren zu ihrer Herstellung und diese enthaltende antibakterielle Mittel

Die vorliegende Erfindung betrifft neue 8-Cyano-1-cyclopropyl-1,4-dihydro-4-oxo-3-chinolincarbonsäurederivate, Verfahren zu ihrer Herstellung sowie diese enthaltende antibakterielle Mittel.

Gefunden wurden die neuen 8-Cyano-1-cyclopropyl-1,4-dihydro-4-oxo-3-chinolincarbonsäurederivate der Formel I

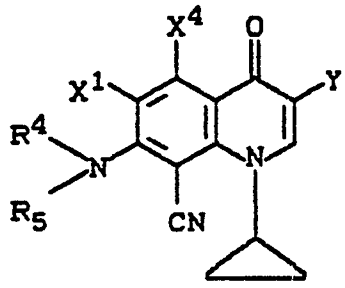

(I)

in welcher

Y eine Carboxylgruppe, eine Nitrilgruppe, eine Estergruppe -COOR$^1$ oder Säureamidgruppe -CONR$^2$ R$^3$ darstellt, wobei R$^1$ für Alkyl, vorzugsweise $C_1$-$C_4$-Alkyl und R$^2$ und R$^3$ unabhängig voneinander für Wasserstoff oder Alkyl, vorzugsweise $C_1$-$C_4$-Alkyl stehen und R$^3$ außerdem gegebenenfalls substituiertes Phenyl sein kann,

X$^1$ für Wasserstoff, Nitro, Alkyl vorzugsweise mit 1-3 Kohlenstoffatomen oder Halogen, bevorzugt Fluor steht,

X$^4$ Wasserstoff, Halogen, vorzugsweise Cl oder F oder Alkyl, bevorzugt mit 1-3 C-Atomen, insbesondere Methyl, sein kann

R$^4$ und R$^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, -$SO_2$-,

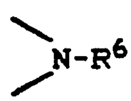 oder 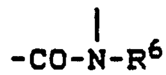

enthalten kann

und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminometyl und Ethylaminomethyl substituiert sein kann, wobei

R$^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest COR$^7$ oder $SO_2R^8$ bedeutet, wobei R$^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Amino, Alkylamino oder Dialkylamino mit 1 bis 5 Kohlenstoffatomen im Alkylteil und

R$^8$ geradkettiges oder verzweigtes Alkyl mit 1 bis 3 Kohlenstoffatomen darstellt, oder die Gruppe

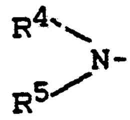

auch für ein Ringsystem der Struktur

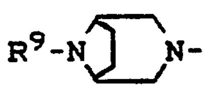 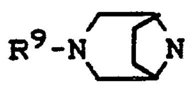 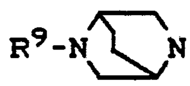 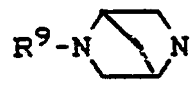

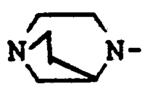 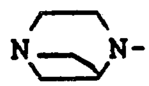

stehen kann, das gegebenenfalls an den Ring-Kohlenstoffen durch Methyl substituiert sein kann, worin $R^9$ für Wasserstoff, Methyl, Ethyl, Hydroxyethyl, Benzyl oder p-Aminobenzyl stehen kann und deren pharmazeutisch verwendbare Hydrate oder Salze, vorzugsweise Alkali-, Erdalkali-, Silber-und Guanidiniumsalze, sowie ihre Ester.

Die Verbindungen weisen eine hohe antibakterielle Wirksamkeit auf. Sie eignen sich daher als Wirkstoffe für die Human-und Veterinärmedizin. Sie können auch als Zwischenprodukte zur Herstellung weiterer Bakterizide verwendet werden.

Bevorzugt sind diejenigen Verbindungen der Formel (I), in denen
Y eine Carboxylgruppe, eine Nitrilgruppe oder eine Estergruppe -COOR$^1$ darstellt, wobei $R^1$ Methyl oder Ethyl ist,
$X^1$ für Fluor steht,
$X^4$ für Wasserstoff steht,
$R^4$ und $R^5$ gemeinsam mit dem Stickstoffatom, an das sie gebunden sind, einen 5-oder 6-gliedrigen heterocyclischen Ring bilden können, der als Ringglied zusätzlich ein Sauerstoffatom oder die Gruppen

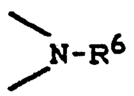 oder 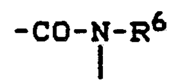

enthalten kann und der gegebenenfalls an den Kohlenstoffatomen ein-bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^6$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest COR$^7$ steht, wobei $R^7$ Wasserstoff oder Alkyl mit ein oder zwei Kohlenstoffatomen bedeutet, oder die Gruppe

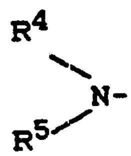

auch für ein Ringsystem der Struktur

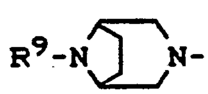 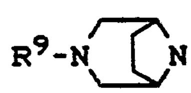 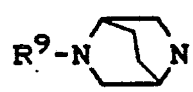

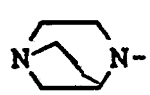 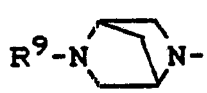 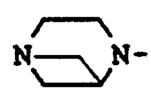

stehen kann, worin
$R^9$ für Wasserstoff und Methyl stehen kann.

Besonders bevorzugt sind Verbindungen der Formel I, in denen
Y eine Carboxylgruppe darstellt,
$X^1$ für Fluor steht,
$X^4$ für Wasserstoff steht,
$R^4$ und $R^5$ gemeinsam mit dem Stickstoffatom, an das sie gebunden sind, einen 5-oder 6-gliedrigen heterocyclischen Ring bilden können, der als Ringglied zusätzlich einen Sauerstoffatom oder die Gruppen

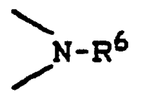 oder 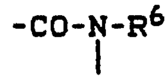

enthalten kann und der gegebenenfalls an den Kohlenstoffatomen ein-bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^6$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest $COR^7$ steht, wobei $R^7$ Wasserstoff oder Alkyl mit ein oder zwei Kohlenstoffatomen bedeutet.

Die erfindungsgemäßen Verbindungen der Formel I erhält man, wenn man Chinoloncarbonsäurederivate der Formel (II)

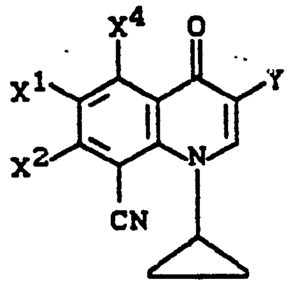

(II)

in der die Reste $X^1$, $X^4$ und Y die oben angegebenen Bedeutungen haben, sowie $X^2$ für Halogen, bevorzugt für Chlor und Fluor, steht,
mit Aminen der Formel III

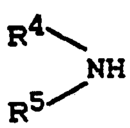

(III)

in welcher
$R^4$ und $R^5$ die oben angegebenen Bedeutungen haben, gegebenenfalls in Gegenwart von Säurebindern umsetzt. (Methode A)

Dieses Verfahren muß nicht notwendigerweise so gestaltet werden, daß $R^4$ und $R^5$ in den Aminen der Formel (III) bereits ihre endgültige Bedeutung haben, die sie in den erfindungsgemäßen Verbindungen der Formel (I) haben. Es können vielmehr auch in einem ersten Schritt Vorstufen zu den Resten $R^4$ und $R^5$ benutzt werden, die dann in einem oder mehreren Folgereaktionsschritten in die endgültige Form von $R^4$ und $R^5$ überführt werden.

So können beispielsweise erfindungsgemäße Verbindungen der Formel (I) erhalten werden, indem man eine 7-(1-(Piperazinyl)-Verbindung der Formel (IV)

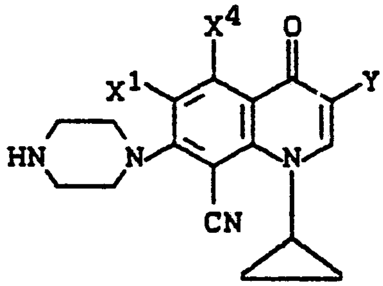

(IV)

in welcher

$X^1$, $X^4$ und Y die oben angegebenen Bedeutungen haben und der Piperazinylrest an den Kohlenstoffatomen in der bei $R^4$ und $R^5$ angegebenen Weise substituiert sein kann, beispielsweise 1-bis 3-fach durch $C_1$-$C_4$-Alkyl, 2-Thienyl oder gegebenenfalls substituiertes Cyclohexyl oder Phenyl mit Verbindungen der Formel (V)

$R^6X$ (V)

in welcher

$R^6$ die oben angegebene Bedeutung hat, jedoch nicht Wasserstoff sein kann, und

X Fluor, Chlor, Brom, Iod, Hydroxy, Acyloxy, Ethoxy, Phenoxy, 4-Nitrophenoxy bedeutet,

gegebenenfalls in Gegenwart von Säurebindern umsetzt. (Methode B).

Bei dieser Reaktionsführung kann also der in der 7-Stellung befindliche Piperazinylrest in einem ersten Reaktionsschritt nach der zuerst genannten Methode eingeführt werden - was zu der bereits erfindungsgemäßen Verbindung (IV) führt - und in einem Folgeschritt dann gewünschte weitere Substituenten eingeführt werden, hier beispielhaft $R^6$.

In einer weiteren Ausgestaltung des erfindungsgemäßen Verfahrens erhält man erfindungsgemäße Verbindungen der Formel (I), z. B. wenn man 7-(1-Piperazinyl)chinoloncarbonsäurederivate der Formel (IV), in welcher der Piperazinylrest an den Kohlenstoffatomen in der bereits genannten Weise, beispielsweise 1-bis 3-fach durch $C_1$-$C_4$-Alkyl, 2-Thienyl oder gegebenenfalls substituiertes Cyclohexyl oder Phenyl substituiert sein kann, mit Michael-Acceptoren der Formel (VI)

$B\text{-}CH=CH_2$ (VI)

in der

B für CN, CO-$R^{10}$ oder $COOR^{11}$ steht, wobei $R^{10}$ für Methyl oder Ethyl und $R^{11}$ für Methyl, Ethyl, n-oder i-Propyl steht,

umsetzt. (Methode C)

Verwendet man beispielsweise bei der Umsetzung nach der erstgenannten Methode 1-Methylpiperazin und 7-Chlor-8-cyano-1-cyclopropyl-6-fluor-1,4-dihydro-4-oxo-3-chinolin carbonsäure als Ausgangsstoffe, so kann der Reaktionsablauf durch folgendes Schema wiedergegeben werden:

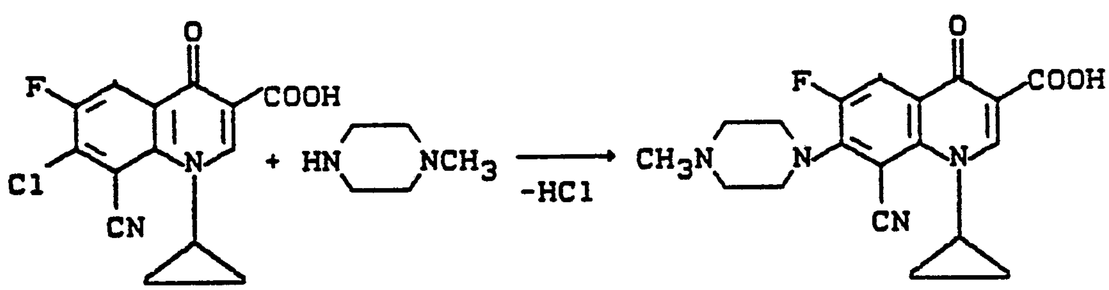


Verwendet man zum Beispiel bei der Umsetzung nach der modifizierten Methode Ethyliodid und 8-Cyano-1-cyclopropyl-6-fluor-1,4-dihydro-4-oxo-7(1-piperazinyl)-3-chinolincarbonsäure als Ausgangsstoffe, so kann der Reaktionsablauf durch folgendes Formelschema wiedergegeben werden:

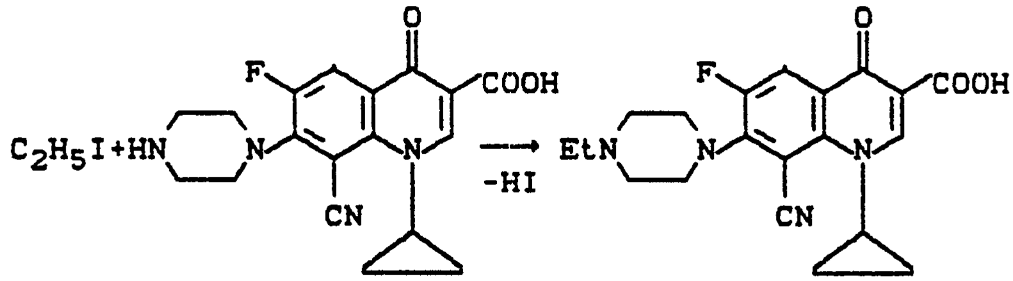


Die Umsetzung mit Michael-Acceptoren mit beispielsweise 8-Cyano-1-cyclopropyl-6-fluor-1,4-dihydro-4-oxo-7-(1-piperazinyl)-3-chinolincarbonsäure und Methylvinylketon als Ausgangsstoffen kann durch folgendes Formelschema wiedergegeben werden:

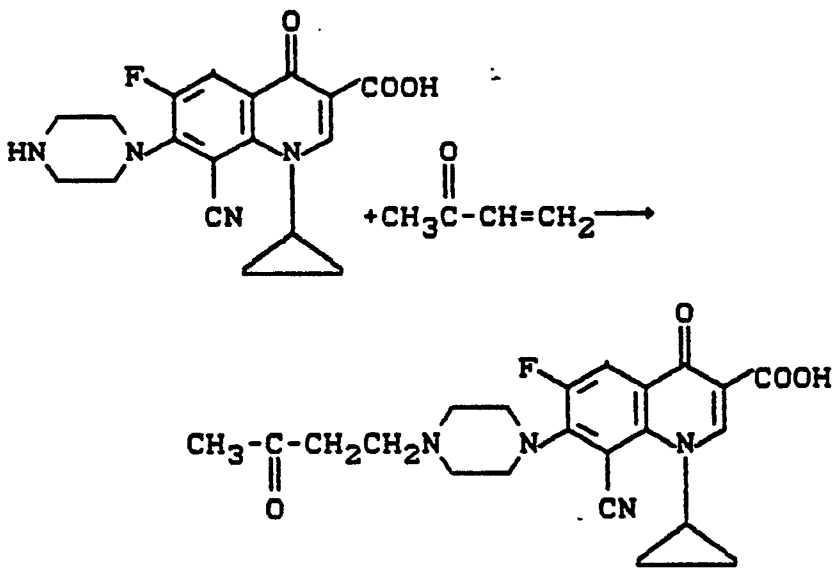

Die als Ausgangsstoffe im erfindungsgemäßen Verfahren verwendbaren Chinoloncarbonsäuren der Formel (II) können gemäß folgendem Schema hergestellt werden. (Verfahren 1)

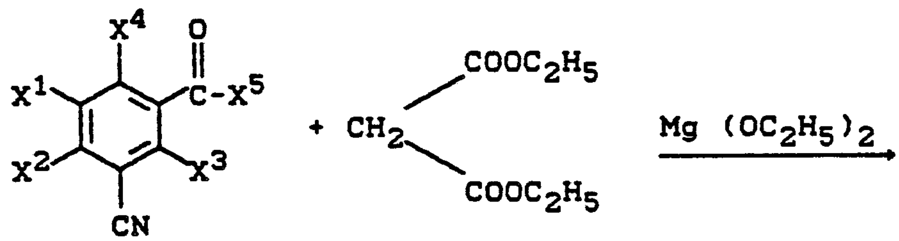


(1) (2)

$X^3$ = F, Cl, $NO_2$
$X^1$, $X^2$, $X^4$ wie oben angegeben
$X^5$ = Cl, Br, F

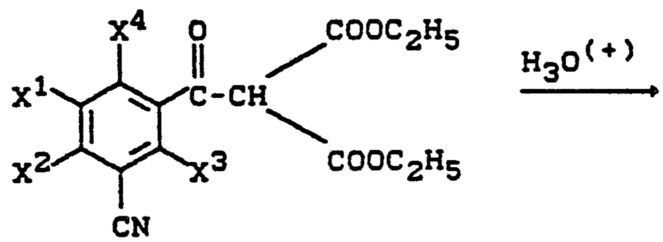


(3)

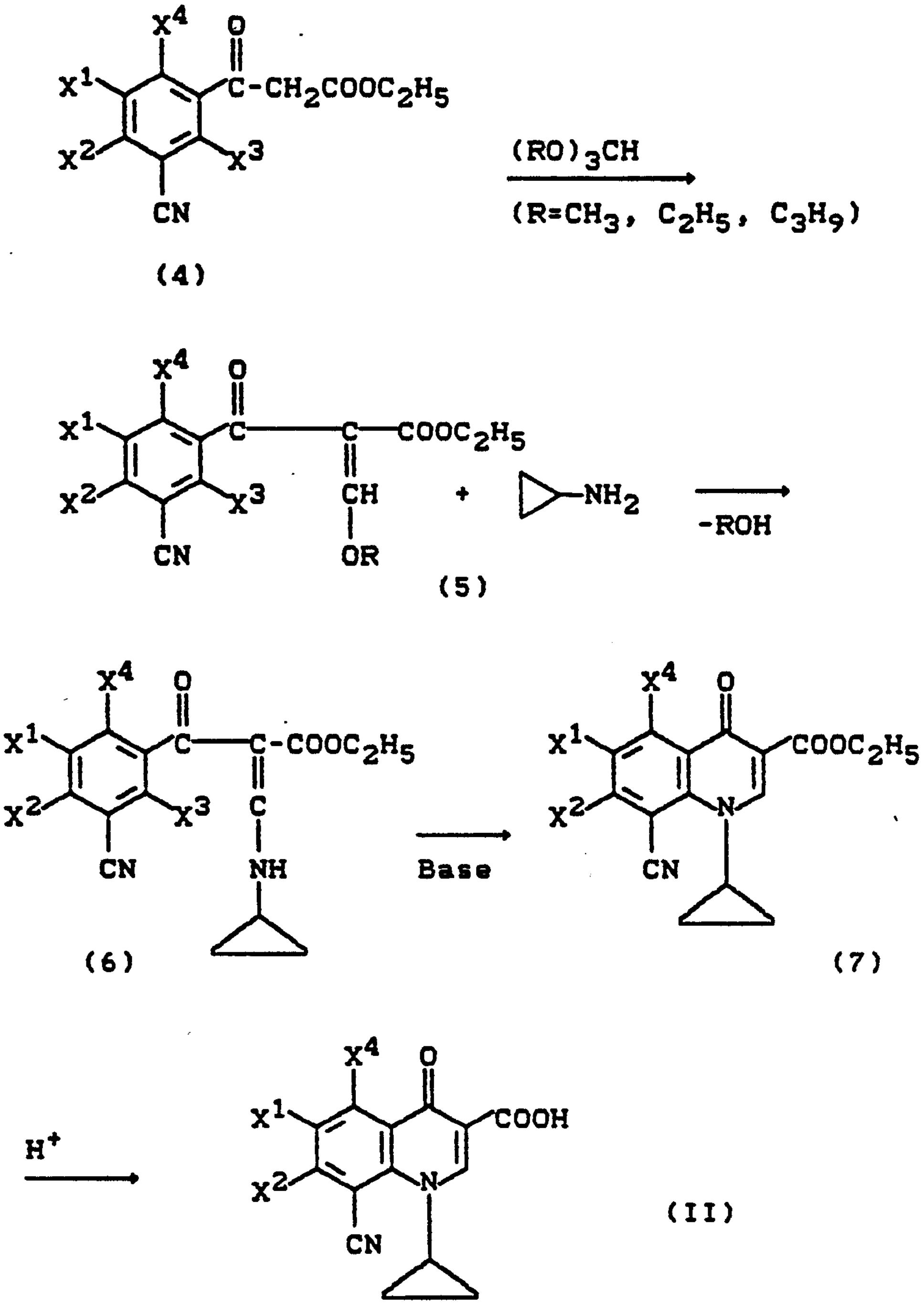


(4)

(5)

(6)

(7)

(II)

Danach wird Malonsäurediethylester (2) in Gegenwart von Magnesiumethylat mit dem entsprechenden Benzoylfluorid bzw. -chlorid (1) zum Benzoylmalonester (3) acyliert (Organicum, 3. Aufl. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Benzoylessigsäureethylester (4), der mit Orthoameisensäuretriethylester/Acetanhydrid in den 3-Ethoxyacrylsäureethylester (5) übergeht. Die Umsetzung von (5) mit Cyclopropylamin in einem Lösungsmittel, wie z. B. Methylenchlorid, Alkohol, Chloroform, Cyclohexan oder Toluol führt in leicht exothermer Reaktion zum gewünschten Zwischenprodukt (6).

Die Cyclisierungsreaktionen (6)→(7) werden in einem Temperaturbereich von etwa 60 bis 300°C, bevorzugt 80 bis 180°C, durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-butanolat, Butyl-lithium, Lithiumphenyl, Phenylmagnesiumbromid, Natriummethylat, Natriumhydrid, Natrium-oder Kaliumcarbonat und besonders bevorzugt Kalium-oder Natrium-fluorid in Betracht. Es kann von Nutzen sein, einen Überschuß von 10 Mol-% an Base einzusetzen.

Die im letzten Schritt erfolgende Hydrolyse der Ester (7) zu den entsprechenden Carbonsäuren kann unter den üblichen sauren oder basischen Bedingungen erfolgen.

Das als Ausgangsstoff für diesen Syntheseweg verwendete 2,4-Dichlor-3-cyano-5-fluorbenzoylchlorid ist wie folgt erhältlich:

a) 2,4-Dichlor-5-fluor-3-nitro-benzoesäure

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45-50°C steigt. Dann wird noch 3 Stunden auf 90-100°C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml $H_2O$ versetzt. Der Niederschlag wird kalt abgesaugt, mit $CH_3OH/H_2O$ gewaschen und im Vakuum bei 80°C getrocknet. Es werden 21,2 g rohe 2,4-Dichlor-5-fluor-3-nitro-benzoesäure erhalten. Sie ist genügend rein für die weiteren Umsetzungen.
Schmelzpunkt: 192°C (aus Toluol-Petrolether).

b) 3-Amino-2,3-dichlor-5-fluor-benzoesäure

254 g (1 mol) 2,4-Dichlor-5-fluor-3-nitro-benzoesäure werden in 1,8 1 Ethanol in Gegenwart von 60 g Raney-Nickel bei 11-20°C und 10 bar Wasserstoff hydriert, filtriert und im Vakuum eingeengt. Der teigige Rückstand wird mit Wasser verknetet, das auskristallisierte Produkt abgesaugt, mit Wasser gewaschen und getrocknet. Ausbeute: 197 g (88 % der Theorie)
Schmelzpunkt: 175-177°C, aus Toluol: 184-187°C

c) 2,4-Dichlor-3-cyano-5-fluor-benzoesäure

56 g 3-Amino-2,4-dichlor-5-fluor-benzoesäure werden in 700 ml halbkonzentrierter Schwefelsäure durch Zugabe von 2,5 molarer $NaNO_2$-Lösung bei 0-5°C diazotiert. Überschüssiges Nitrit wird durch Zugabe von Harnstoff zerstört. Anschließend tropft man die Diazoniumsalzlösung bei 0°C zu einer Mischung von 27 g CuCN und 200 ml 4,5 molarer NaCN-Lösung. Nach Ende des Zutropfens wird eine Stunde auf 80°C erwärmt. Danach kühlt man ab, isoliert den Feststoff und trocknet ihn. Der Feststoff (64,3 g) wird mit Toluol ausgekocht. Man filtriert vom Unlöslichen ab und dampft das Lösungsmittel weitgehend ab. Beim Abkühlen kristallisiert die Säure aus.
Ausbeute: 44 g
Schmelzpunkt: 188-190°C; nochmaliges Umkristallisieren aus Toluol ergibt einen Schmelzpunkt 203-205°C.

d) 2,4-Dichlor-3-cyano-5-fluor-benzoesäurechlorid

25 g 2,4-Dichlor-3-cyano-5-fluor-benzoesäure und 30 ml Thionylchlorid werden solange gekocht, bis die Gasentwicklung beendet ist. Anschließend zieht man das überschüssige Thionylchlorid im Vakuum ab. Es bleiben 25,6 g Säurechlorid vom Schmelzpunkt 69-72°C zurück.

Die als Ausgangsstoffe verwendeten Amine (III) sind bekannt oder können nach literaturbekannten Verfahren erhalten werden, US 4 166 188, J. Med. Chem. 26, 1116 (1983). Durch katalytische Hydrierung werden aus den 2-Arylpiperazinen der entsprechenden 2-Cyclohexyl-piperazine erhalten: z. B. 2-Cyclohexylpiperazin (wachsartig, Schmelzpunkt 71-73°C). Als Beispiele seien genannt:

Morpholin, Piperidin, Thiomorpholin, Pyrrolidon, Piperazin, N-Methylpiperazin, N-Ethylpiperazin, N-(2-Hydroxyethyl)-piperazin, N-Formylpiperazin, 2-Methylpiperazin, 1,2-Dimethylpiperazin, cis-und trans-2,5-Dimethylpiperazin, 2-Propylpiperazin, 2-Isopropylpiperazin, 2-Isobutylpiperazin, 2-Piperazinon, 1-Methyl-2-piperazinon, 1-Ethyl-2-piperazinon, 2-Cyclohexyl-1-piperazin, 2-Phenylpiperazin, 2-(4-Chlorphenyl)-piperazin, 2-(4-Fluorphenyl)-piperazin, 2-(4-Brom-phenyl)-piperazin, 2-(4-Methylphenyl)-piperazin, 2-(4-Biphenyl)-piperazin, 2-(4-Methylphenyl)-piperazin, 2-(4-Benzyloxyphenyl)-piperazin, 2-(4-Hydroxyphenyl)-piperazin, 2-(4-Nitrophenyl)-piperazin, 2-(3-Nitrophenyl)-piperazin, 2-(4-Piperidino-phenyl)-piperazin, 2-(3,4-Dimethoxyphenyl)-piperazin, 2-(3,4,5-Trimethoxyphenyl)-piperazin, 2-(3-dimethoxy-6-methyl)-piperazin, 2-(2-Thienyl)-piperazin, 3-Amino-pyrrolidin, 2,5-Diazabicyclo [2.2.1] heptan-Dihydrochlorid, 2-Methyl-2,5-diazabicyclo [2.2.1] heptan-Dihydrochlorid, 8-Methyl-3,8-diazabicyclo [2.2.1] octan-Dihydrochlorid, 3-Methyl-3,8-diazabicyclo [2.2.1] octan-Dihydrochlorid, 3-(4-Aminobenzyl)-3,8-diazbicyclo [3.2.1] octan, 3-Ethyl-3,8-diazabicyclo [3.2.1] octan, 3-Benzyl-3,8-diazabicyclo [3.2.1]octan, 2-Methyl 2,5-diazabicyclo [2.2.2] octan-Dihydrochlorid.

Die als Ausgangsstoffe verwendeten Verbindungen der Formel (V) sind bekannt. Als Beispiele seien genannt:

Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, n-Pentylchlorid, 3-Methylbutylchlorid, n-Hexylbromid. Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4-nitro-phenylester, N-(tert.-Butoxycarbonyl)-L-valin-4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Ameisensäure.

Die erfindungsgemäß verwendbaren Verbindungen der Formel (VII) sind bekannt. Als Beispiele seien genannt:

Acrylnitril, Methylvinylketon, Acrylsäuremethylester, Acrylsäureethylester.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen organischen und anorganischen Säurebindemittel verwendet werden. Hierzu gehören vorzugsweise die Alkalihydroxide, Alkalicarbonate, organischen Amine und Amidine. Als besonders geeignet seien im einzelnen genannt:Triethylamin, 1,4-Diaza-bicyclo [2.2.2]octan (DABCO), 1,8-Diazabicyclo [5.4.0]-undec-7en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise mit einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amide und Amidine. Als besonders geeignet seien im einzelnen genannt: Triethylamin, 1,4-Diazabicyclo [2.2.2]-octan (DABCO) oder 1,8-Diazabicyclo-[5,4,0]undec-7-en (DBU).

Die Reaktionstemperaturen können in einem größeren Bereich variiert werden. Im allgemeinen arbeitet man zwischen etwa 20 und etwa 180°C, vorzugsweise zwischen 40 und 110°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol, der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20°C und etwa 150°C, vorzugsweise zwischen 50°C und 100°C.

Die Umsetzung kann bei Normaldurck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der

Verbindung (IV) 1 bis 5 Mol, vorzugsweise 1 bis 2 Mol, der Verbindung (VI) ein.

Außer in den Beispielen aufgeführten Verbindungen seien als neue Wirkstoffe im einzelnen genannt:

8-Cyan-1-cyclopropyl-6-fluor-1,4-dihydro-7-(4-morpholinyl)-4-oxo-3-chinolincarbonsäure, 8-Cyan-1-cyclopropyl-6-fluor-1,4-dihydro-4-oxo-7-(3-Phenyl-1-piperazinyl)-3-chinolincarbonsäure, 6-Chlor-8-cyan-1-cyclopropyl-1,4-dihydro-4-oxo-7(1-piperazinyl)-3-chinolincarbonsäure, 8-Cyan-1-cyclopropyl-6-fluor-1,4-dihydro-oxo(4-thiomorpholinyl)-3-chinolincarbonsäure, 8-Cyan-1-cyclopropyl-6-fluor-1,4-dihydro-7-(1-piperidinyl)-4-oxo-3-chinolincarbonsäure, 7-(3-Amino-1-pyrrolidinyl)-8-Cyan-1-cyclopropyl-1,4-dihydro-4-oxo-3-chinolincarbonsäure, 8-Cyan-1-cyclopropyl-6-fluor-1,4-dihydro-4-oxo-7(1-piperidinyl)-3-chinolincarbonsäure, 8-Cyan-1-cyclopropyl-7-(3-ethylamino-1-pyrrolidinyl)-6-fluor-1,4-dihydro-4-oxo-3-chinolincarbonsäure, 8-Cyan-1-cyclopropyl-7(3-ethylaminomethyl-1-pyrrolidinyl)-6-fluor-1,4-dihydro-4-oxo-3-chinolincarbonsäure, 8-Cyan-1-cyclopropyl-7(-2,5-diazabicyclo[2.2.1]oct-5-yl)-6-fluor-1,4-dihydro-4-oxo-3-chinolincarbonsäure, 8-Cyan-1-cyclopropyl-7-(3,8-diazabicyclo[3.2.1]oct-3-yl)-6-fluor-1,4-dihydro-4-oxo-3-chinolincarbonsäure, 8-Cyan-1-cyclopropyl-6-fluor-1,4-dihydro-7(5-methyl-2,5-diazabicyclo[2.2.1]hept-2-yl)4-oxo-3-chinolincarbonsäure, 6-Chlor-8-cyan-1-cyclopropyl-7-(3,8-diazabicyclo[3.2.1]oct-3-yl]-1,4-dihydro-4-oxo-3-chinolincarbonsäure, 6-Chlor-8-cyan-1-cyclopropyl-1,4-dihydro-7-(5-methyl-2,5-diazabicyclo[2.2.1]hept-2-yl)-4-oxo-3-chinolincarbonsäure, 8-Cyan-1-cyclopropyl-1,4-dihydro-7-(5-methyl-2,5-diazabicyclo[2.2.1]hept-2-yl)-6-nitro-4-oxo-3-chinolincarbonsäure, 8-Cyan-1-cyclopropyl-1,4-dihydro-6-nitro-4-oxo-7(1-piperazinyl)-3-chinolincarbonsäure, 8-Cyan-1-cyclopropyl-1,4-dihydro-7(3-methyl-1-piperazinyl)-6-nitro-4-oxo-3-chinolincarbonsäure, 8-Cyan-1-cyclopropyl-1,4-dihydro-6-nitro-4-oxo-7-(3-Phenyl-1-piperazinyl)-3-chinolincarbonsäure, 6-(3-Amino-1-pyrrolidinyl)-8-cyan-1-cyclopropyl-1,4-dihydro-6-nitro-4-oxo-3-chinolincarbonsäure, 8-Cyan-1-cyclopropyl-5,6-difluor-1,4-dihydro-4-oxo-7(1-piperazinyl)-3-chinolin carbonsäure, 8-Cyan-1-cyclopropyl-5,6-difluor-1,4-dihydro-7-(4-methyl-1-piperazinyl)-4-oxo-3-chinolincarbonsäure, 5-Chlor-8-cyan-1-cyclopropyl-6-fluor-1,4-dihydro-7-(4-methyl-1-piperazinyl)-4-oxo-3-chinolincarbonsäure, 5-Chlor-8-cyan-1-cyclopropyl-7-(3,8-diazabicyclo[3.2.1]oct-3-yl)-6-fluor-1,4-dihydro-4-oxo-3-chinolincarbonsäure, 5-Chlor-8-cyan-1-cyclopropyl-7(3-ethylamine-1-pyrrolidinyl)-6-fluor-1,4-dihydro-4-oxo-3-chinolincarbonsäure, 8-Cyan-1-cyclopropyl-6-fluor-1,4-dihydro-5-methyl-4-oxo-7-(1-piperazinyl)-3-chinolincarbonsäure, 7-(3-Amino-1-pyrrolidinyl)-8-cyan-1-cyclopropyl-6-fluor-1,4-dihydro-5-methyl-4-oxo-3-chinolincarbonsäure, 8-Cyan-1-cyclopropyl-6-fluor-1,4-dihydro-5-methyl-4-oxo-7-(3-Phenyl-1-piperazinyl)-3-chinolincarbonsäure, 8-Cyan-1-cyclopropyl-7-(3,8-diazabicyclo[3.2.1]oct-3-yl)-6-fluor-1,4-dihydro-5-methyl-4-oxo-3-chinolincarbonsäure, 8-Cyan-1-cyclopropyl-7-(3-ethylamino-1-pyrrolidinyl)-6-fluor-1,4-dihydro-5-methyl-4-oxo-3-chinolincarbonsäure, 8-Cyan-1-cyclopropyl-1,4-dihydro-5,6-dimethyl-4-oxo-7-(1-piperazinyl)-3-chinolincarbonsäure, 8-Cyan-1-cyclopropyl-1,4-dihydro-5,6-dimethyl-4-oxo-7-(1-pyrrolidinyl)-3-chinolincarbonsäure.

Die folgenden Beispiele erläutern die Erfindung.

Beispiel 1

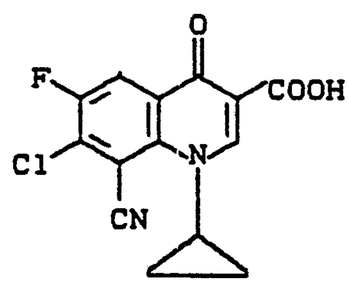

7-Chlor-8-cyano-1-cyclopropyl-6-fluor-1,4-dihydro-4-oxo-3-chinolincarbonsäure

1,2 g Magnesiumspäne werden in 3 ml Ethanol und 0,3 ml $CCl_4$ verrührt und nach begonnener Reaktion bei 50-60°C tropfenweise mit 7 g Malonsäurediethylester in 5 ml EtOH und 18 ml Toluol versetzt. Man rührt 1 Stunde bei dieser Temperatur nach, kühlt auf -5° bis -10°C, tropft eine Lösung von 11 g 2,4-Dichlor-3-cyano-5-fluor-benzoesäurechlorid in 5 ml Toluol zu, rührt noch 1 Stunde bei 0°C und läßt über Nach bei Raumtemperatur stehen. Danach wird abgekühlt und mit 20 ml Wasser und 3 ml konzentrierter $H_2SO_4$ versetzt. Die organische Phase wird abgetrennt, die wäßrige mit Toluol extrahiert, die vereinigten organischen Phasen mit gesättigter NaCl-Lösung gewaschen, mit Natriumsulfat getrocknet und eingeengt.

Der Rückstand (16,7 g) wird mit 20 ml Wasser und 0,35 g p-Toluolsulfonsäure 4,5 Stunden gekocht. Anschließend extrahiert man mit $CH_2Cl_2$, wäscht die organische Phase mit Natriumchloridlösung, trocknet

über Natriumsulfat und engt ein. Es bleiben 11,6 g Rückstand.

Der Rückstand wird zusammen mit 8,7 g Orthoameisensäureester und 9,8 g Essigsäureanhydrid zwei Stunden auf 150°C erhitzt und anschließend wird bei 120-130°C zuerst unter Normaldruck, dann im Hochvakuum eingeengt. Es werden 12,7 g 2(2,4-Dichlor-3-cyan-5-fluorbenzoyl)-3-ethoxyacrylsäureethylester als Öl erhalten.

Die 12,7 g dieser Verbindung werden in 30 ml Ethanol unter Eiskühlung mit 2,5 g Cyclopropylamin versetzt und 2 Stunden bei Raumtemperatur gerührt. Danach wird mit 30 ml Wasser verrührt, in Eis gekühlt, der ausgefallene Feststoff abgetrennt, mit Wasser gewaschen und getrocknet.

Es werden 11,8 g 2(2,4-Dichlor-3-cyano-5-fluor-benzoyl)-3-cyclopropylamino-acrylsäureethylester vom Schmelzpunkt 65-67°C erhalten.

41,2 g dieser Verbindung und 13,6 g KOtBu werden in 500 ml Dioxan 24 Stunden bei Raumtemperatur gerührt. Anschließend wird Wasser zugegeben und mit $CH_2Cl_2$ extrahiert.

Die organische Phase wird gewaschen, über Natriumsulfat getrocknet und eingeengt. Der verbleibende Rückstand wird mit Isopropanol verrührt. Der anfallende Feststoff wird isoliert und getrocknet. Es werden 18,0 g 7-Chlor-8-cyano-1-cyclopropyl-6-fluor-1,4-dihydro-4-oxo-3-chinolincarbonsäureethylester vom Schmelzpunkt 162-65°C erhalten.

1 g dieser Verbindung werden zusammen mit 3,5 ml Essigsäure, 3 ml Wasser und 0,3 ml Schwefelsäure 4 Stunden auf 140-5°C erhitzt. Anschließend wird mit Wasser verdünnt und der Feststoff isoliert. Es werden 0,7 g der Titelverbindung vom Schmelzpunkt 281-282°C erhalten.

Beispiel 2

8-Cyano-1-cyclopropyl-6-fluor-1,4-dihydro-4-oxo-7(1-piperazinyl)-3-chinolincarbonsäure

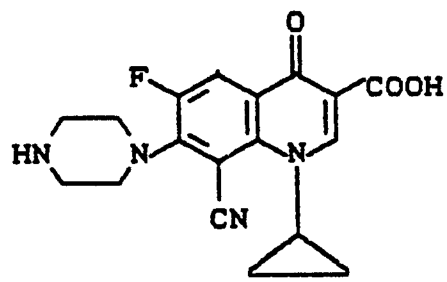

0.5 g Produkt aus Beispiel 1 und 0,42 g Piperazin werden in 8 ml Dioxan 3 Stunden gekocht. Danach wird im Vakuum eingeengt und der Rückstand mit 8 ml Wasser versetzt. Die entstandene Lösung wird mit HCl neutral gestellt, der ausgefallene Feststoff isoliert, gewaschen und getrocknet.

Ausbeute : 0,4 g der Titelverbindung

Schmelzpunkt: > 300°C

Beispiel 3

8-Cyano-1-cyclopropyl-6-fluor-1,4-dihydro-7(4-methyl-1-piperazinyl)-4-oxo-3-chinolincarbonsäure

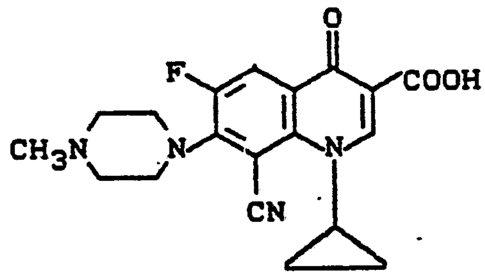

0,5 g Produkt aus Beispiel 1 und 0,48 g N-Methyl-piperazin werden in 8 ml Dioxan 3 Stunden gekocht. Danach wird im Vakuum eingeengt, der Rückstand mit Wasser versetzt und die entstehende Lösung neutral gestellt. Der ausgefallene Feststoff wird isoliert, gewaschen und getrocknet.
Ausbeute : 0,5 g der Titelverbindung
Schmelzpunkt: 264-265°C

Beispiel 4

8-Cyano-1-cyclopropyl-7(4-ethyl-1-piperazinyl)-6-fluor-1,4-dihydro-4-oxo-3-chinolincarbonsäure

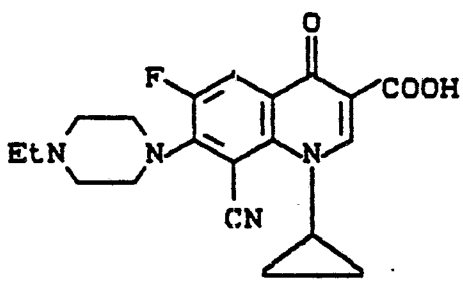

0,5 g Produkt aus Beispiel 1 und 0,55 g N-Ethyl-piperazin werden in 8 ml Dioxan 3 Stunden gekocht. Anschließend wird im Vakuum eingeengt. Der Rückstand wird mit Wasser aufgenommen und neutral gestellt. Man extrahiert mit Methylenchlorid, trennt die organische Phase ab, trocknet sie über Natriumsulfat und engt ein. Es bleiben 0,5 g der Titelverbindung mit einem Schmelzpunkt > 300°C zurück.

Beispiel 5

8-Cyano-1-cyclopropyl-6-fluor-1,4-dihydro-7-(3-methyl-1-piperazinyl)-4-oxo-3-chinolincarbonsäure

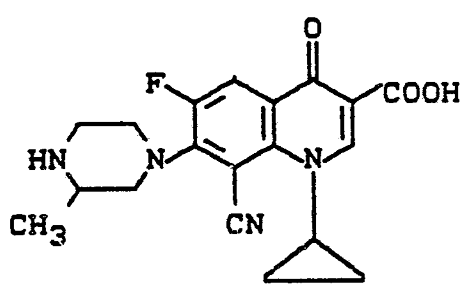

0,5 g Produkt aus Beispiel 1 und 0,48 g 2-Methyl-piperazin werden in 8 ml Dioxan 3 Stunden gekocht. Anschließend wird im Vakuum eingeengt und der Rückstand mit Wasser versetzt.

Man gibt 1n-HCl zu, bis die Lösung neutral ist. Der ausgefallene Feststoff wird abgenutscht und getrocknet. Es werden 0,5 g der Titelverbindung mit einem Schmelzpunkt > 300°C erhalten.

Beispiel 6

8-Cyano-1-cyclopropyl-6-fluor-1,4-dihydro-4-oxo-7-(1-pyrrolidinyl)-3-chinolincarbonsäure

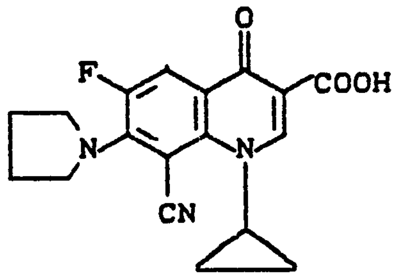

3,05 g Produkt aus Beispiel 1 und 2,5 ml Pyrrolidin werden in 50 ml Dioxan 3 Stunden gekocht. Man engt im Vakuum ein und nimmt den Rückstand in Wasser auf. Es wird mit 1n-HCl neutral gestellt. Der ausgefallene Feststoff wird abgenutscht, getrocknet und mit Acetonitril verrührt. Es werden 1,8 g der Titelverbindung mit dem Schmelzpunkt 274-6°C erhalten.

Beispiel für eine erfindungsgemäße Tablette

Jede Tablette enthält:

| | |
|---|---|
| Verbindung des Beispiels 5 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykol (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephri tis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund and Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegvserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugt pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Workstoffe nur oder bevorzugt in einem bestimmten Teil des Intesti naltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lö sungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Workstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit-und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber-und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten,

Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human-als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

## Ansprüche

1. 8-Cyano-1-cyclopropyl-1,4-dihydro-4-oxo-3-chinolincarbonsäurederivate der Formel I

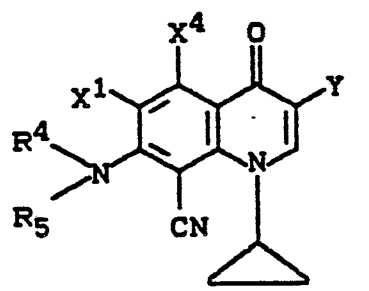

(I)

in welcher
Y eine Carboxylgruppe, eine Nitrilgruppe, eine Estergruppe $-COOR^1$ oder Säureamidgruppe $-CONR^2 R^3$ darstellt, wobei $R^1$ für Alkyl, vorzugsweise $C_1$-$C_4$-Alkyl und $R^2$ und $R^3$ unabhängig voneinander für Wasserstoff oder Alkyl, vorzugsweise $C_1$-$C_4$-Alkyl stehen und $R^3$ außerdem gegebenenfalls substituiertes Phenyl sein kann,
$X^1$ für Wasserstoff, Nitro, Alkyl vorzugsweise mit 1-3 Kohlenstoffatomen oder Halogen, bevorzugt Fluor steht,
$X^4$ Wasserstoff, Halogen, vorzugsweise Cl oder F oder Alkyl, bevorzugt mit 1-3 C-Atomen, insbesondere Methyl, sein kann
$R^4$ und $R^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, $-SO_2-$,

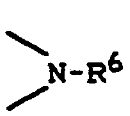 oder 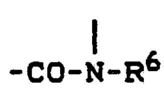

enthalten kann und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl 2-Thienyl, Hydroxy, Alkoxy mit

1 bis 3 Kohlenstoffatomen, Amino, Methylamino, Ethylamino, Aminomethyl, Methylaminometyl und Ethylaminomethyl substituiert sein kann, wobei

$R^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest $COR^7$ oder $SO_2R^8$ bedeutet, wobei $R^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Amino, Alkylamino oder Dialkylamino mit 1 bis 5 Kohlenstoffatomen im Alkylteil und

$R^8$ geradkettiges oder verzweigtes Alkyl mit 1 bis 3 Kohlenstoffatomen darstellt, oder die Gruppe

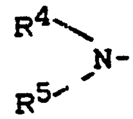

auch für ein Ringsystem der Struktur

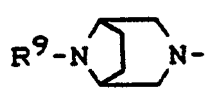 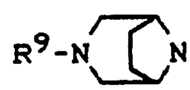 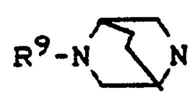 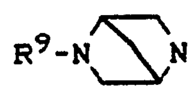

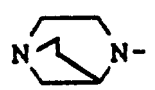 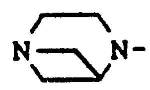

stehen kann, das gegebenenfalls an den Ring-Kohlenstoffen durch Methyl substituiert sein kann, worin

$R^9$ für Wasserstoff, Methyl, Ethyl, Hydroxyethyl, Benzyl oder p-Aminobenzyl stehen kann und

deren pharmazeutisch verwendbare Hydrate oder Salze, vorzugsweise Alkali-, Erdalkali-, Silber-und Guanidiniumsaalze, sowie ihre Ester.

2. Verbindungen der Formel I nach Anspruch 1, in denen

Y eine Carboxylgruppe, eine Nitrilgruppe oder eine Estergruppe $-COOR^1$ darstellt, wobei $R^1$ Methyl oder Ethyl ist,

$X^1$ für Fluor steht,

$X^4$ für Wasserstoff steht,

$R^4$ und $R^5$ gemeinsam mit dem Stickstoffatom, an das sie gebunden sind, einen 5-oder 6-gliedrigen heterocyclischen Ring bilden können, der als Ringglied zusätzlich ein Sauerstoffatom oder die Gruppen

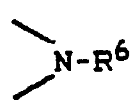 oder 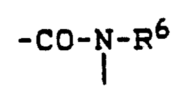

enthalten kann und der gegebenenfalls an den Kohlenstoffatomen ein-bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^6$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest $COR^7$ steht, wobei $R^7$ Wasserstoff oder Alkyl mit ein oder zwei Kohlenstoffatomen bedeutet, oder die Gruppe

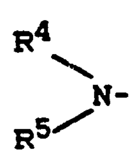

auch für ein Ringsystem der Struktur

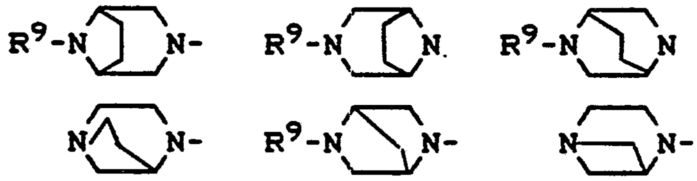

stehen kann, worin

$R^9$ für Wasserstoff und Methyl stehen kann,

3. Verbindungen der Formel I nach Anspruch 1, in denen

Y eine Carboxylgruppe darstellt,

$X^1$ für Fluor steht,

$X^4$ für Wasserstoff steht,

$R^4$ und $R^5$ gemeinsam mit dem Stickstoffatom, an das sie gebunden sind, einen 5-oder 6-gliedrigen heterocyclischen Ring bilden können, der als Ringglied zusätzlich einen Sauerstoffatom oder die Gruppen

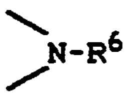 oder 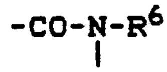

enthalten kann und der gegebenenfalls an den Kohlenstoffatomen ein-bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^6$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, eine Oxalkylrest mit bis zu 4 Kohlenstoffatomen sowie einen Rest $COR^7$ steht, wobei $R^7$ Wasserstoff oder Alkyl mit ein oder zwei Kohlenstoffatomen bedeutet.

4. 8-Cyano-1-cyclopropyl-1,4-dihydro-4-oxo-3-chinolincarbonsäurederivate der Formel I

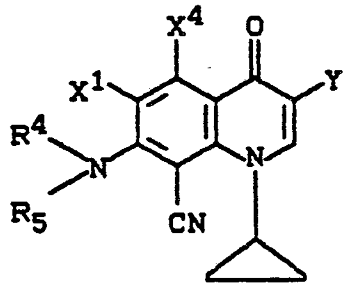

(I)

in welcher

Y eine Carboxylgruppe, eine Nitrilgruppe, eine Estergruppe $-COOR^1$ oder Säureamidgruppe $-CONR^2R^3$ darstellt, wobei $R^1$ für Alkyl, vorzugsweise $C_1$-$C_4$-Alkyl und $R^2$ und $R^3$ unabhängig voneinander für Wasserstoff oder Alkyl, vorzugsweise $C_1$-$C_4$-Alkyl stehen und $R^3$ außerdem gegebenenfalls substituiertes Phenyl sein kann,

$X^1$ für Wasserstoff, Nitro, Alkyl vorzugsweise mit 1-3 Kohlenstoffatomen oder Halogen, bevorzugt Fluor steht,

$X^4$ Wasserstoff, Halogen, vorzugsweise Cl oder F oder Alkyl, bevorzugt mit 1-3 C-Atomen, insbesondere Methyl, sein kann

$R^4$ und $R^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, $-SO_2-$,

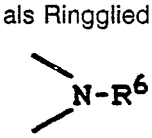 oder 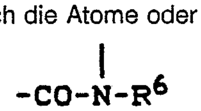

enthalten kann und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminometyl und Ethylaminomethyl substituiert sein kann, wobei

$R^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest $COR^7$ oder $SO_2R^8$ bedeutet, wobei $R^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Amino, Alkylamino oder Dialkylamino mit 1 bis 5 Kohlenstoffatomen im Alkylteil und

$R^8$ geradkettiges oder verzweigtes Alkyl mit 1 bis 3 Kohlenstoffatomen darstellt, oder die Gruppe

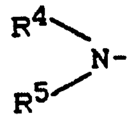

auch für ein Ringsystem der Struktur

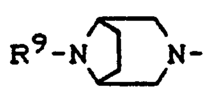
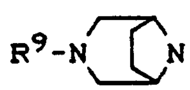
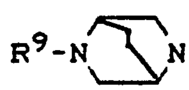
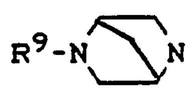
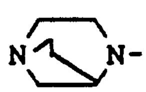
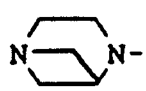

stehen kann, das gegebenenfalls an den Ring-Kohlenstoffen durch Methyl substituiert sein kann, worin

$R^9$ für Wasserstoff, Methyl, Ethyl, Hydroxyethyl, Benzyl oder p-Aminobenzyl stehen kann und deren pharmazeutisch verwendbare Hydrate oder Salze, vorzugsweise Alkali-, Erdalkali-, Silber-und Guanidiniumsalze, sowie ihre Ester, zur Anwendung in einem Verfahren zur therapeutischen Behandlung des menschlichen und tierischen Körpers.

5. Verfahren zur Herstellung von 8 -Cyano-1-cyclopropyl-1,4-dihydro-4-oxo-3-chinolincarbonsäurederivate der Formel I

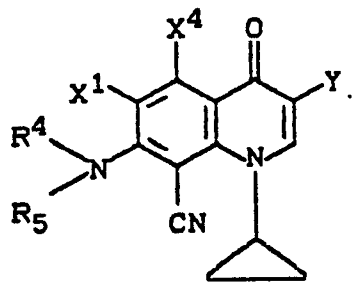

(I)

in welcher

Y eine Carboxylgruppe, eine Nitrilgruppe, eine Estergruppe $-COOR^1$ oder Säureamidgruppe $-CONR^2R^3$ darstellt, wobei $R^1$ für Alkyl, vorzugsweise $C_1$-$C_4$-Alkyl und $R^2$ und $R^3$ unabhängig voneinander für Wasserstoff oder Alkyl, vorzugsweise $C_1$-$C_4$-Alkyl stehen und $R^3$ außerdem gegebenenfalls substituiertes Phenyl sein kann,

$X^1$ für Wasserstoff, Nitro, Alkyl vorzugsweise mit 1-3 Kohlenstoffatomen oder Halogen, bevorzugt Fluor steht,

$X^4$ Wasserstoff, Halogen, vorzugsweise Cl oder F oder Alkyl, bevorzugt mit 1-3 C-Atomen, insbesondere

Methyl, sein kann
$R^4$ und $R^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, $-SO_2-$,

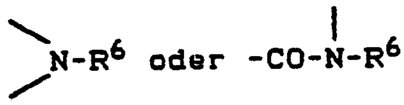

enthalten kann und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminometyl und Ethylaminomethyl substituiert sein kann, wobei
$R^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest $COR^7$ oder $SO_2R^8$ bedeutet, wobei $R^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Amino, Alkylamino oder Dialkylamino mit 1 bis 5 Kohlenstoffatomen im Alkylteil und
$R^8$ geradkettiges oder verzweigtes Alkyl mit 1 bis 3 Kohlenstoffatomen darstellt, oder die Gruppe

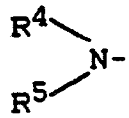

auch für ein Ringsystem der Struktur

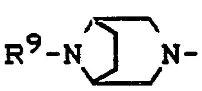
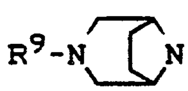
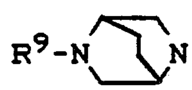
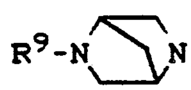
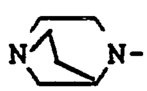
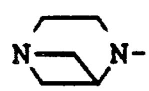

stehen kann, das gegebenenfalls an den Ring-Kohlenstoffen durch Methyl substituiert sein kann, worin
$R^9$ für Wasserstoff, Methyl, Ethyl, Hydroxyethyl, Benzyl oder p-Aminobenzyl stehen kann und
deren pharmazeutisch verwendbaren Hydraten oder Salzen, vorzugsweise Alkali-, Erdalkali-, Silber-und Guanidiniumsalzen, sowie ihren Estern, dadurch gekennzeichnet, daß man Chinoloncarbonsäurederivate der Formel (II)

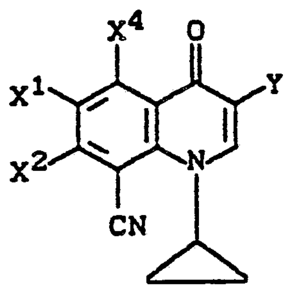

(II)

in der die Reste $X^1$, $X^4$ und Y die oben angegebenen Bedeutungen haben, sowie $X^2$ für Halogen, bevorzugt

für Chlor und Fluor, steht, mit Aminen der Formel III

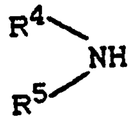

(III)

in welcher $R^4$ und $R^5$ die oben angegebenen Bedeutungen haben, gegebenenfalls in Gegenwart von Säurebindern umsetzt.

6. Arzneimittel, enthaltend 8-Cyano-1-cyclopropyl-1,4-dihydro-4-oxo-3-chinolincarbonsäurederivate der Formel I

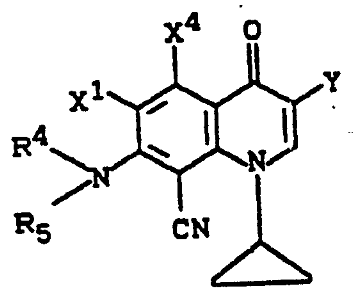

(I)

in welcher Y eine Carboxylgruppe, eine Nitrilgruppe, eine Estergruppe $-COOR^1$ oder Säureamidgruppe $-CONR^2 R^3$ darstellt, wobei $R^1$ für Alkyl, vorzugsweise $C_1$-$C_4$-Alkyl und $R^2$ und $R^3$ unabhängig voneinander für Wasserstoff oder Alkyl, vorzugsweise $C_1$-$C_4$-Alkyl stehen und $R^3$ außerdem gegebenenfalls substituiertes Phenyl sein kann,
$X^1$ für Wasserstoff, Nitro, Alkyl vorzugsweise mit 1-3 Kohlenstoffatomen oder Halogen, bevorzugt Fluor steht,
$X^4$ Wasserstoff, Halogen, vorzugsweise Cl oder F oder Alkyl, bevorzugt mit 1-3 C-Atomen, insbesondere Methyl, sein kann
$R^4$ und $R^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, $-SO_2-$,

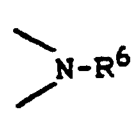 oder 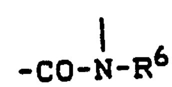

enthalten kann und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminometyl und Ethylaminomethyl substituiert sein kann, wobei
$R^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest $COR^7$ oder $SO_2R^8$ bedeutet, wobei $R^7$ Wasserstoff, gegebenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Amino, Alkylamino oder Dialkylamino mit 1 bis 5 Kohlenstoffatomen im Alkylteil und
$R^8$ geradkettiges oder verzweigtes Alkyl mit 1 bis 3 Kohlenstoffatomen darstellt, oder die Gruppe

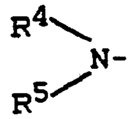

auch für ein Ringsystem der Struktur

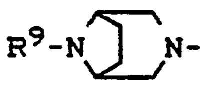 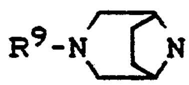 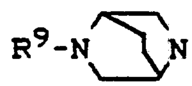 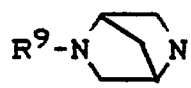

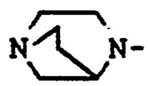 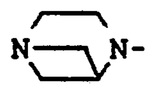

stehen kann, das gegebenenfalls an den Ring-Kohlenstoffen durch Methyl substituiert sein kann, worin $R^9$ für Wasserstoff, Methyl, Ethyl, Hydroxyethyl, Benzyl oder p-Aminobenzyl stehen kann und deren pharmazeutisch verwendbare Hydrate oder Salze, vorzugsweise Alkali-, Erdalkali-, Silber-und Guanidiniumsalze, sowie ihre Ester.

7. Verwendung von 8-Cyano-1-cyclopropyl-1,4-dihydro-4-oxo-3-chinolincarbonsäurederivaten der Formel I

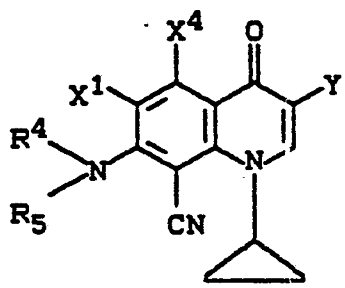

(I) .

in welcher

Y eine Carboxylgruppe, eine Nitrilgruppe, eine Estergruppe $-COOR^1$ oder Säureamidgruppe $-CONR^2R^3$ darstellt, wobei $R^1$ für Alkyl, vorzugsweise $C_1$-$C_4$-Alkyl und $R^2$ und $R^3$ unabhängig voneinander für Wasserstoff oder Alkyl, vorzugsweise $C_1$-$C_4$-Alkyl stehen und $R^3$ außerdem gegebenenfalls substituiertes Phenyl sein kann,

$X^1$ für Wasserstoff, Nitro, Alkyl vorzugsweise mit 1-3 Kohlenstoffatomen oder Halogen, bevorzugt Fluor steht,

$X^4$ Wasserstoff, Halogen, vorzugsweise Cl oder F oder Alkyl, bevorzugt mit 1-3 C-Atomen, insbesondere Methyl, sein kann

$R^4$ und $R^5$ gemeinsam mit dem Stickstoffatom, an das sie gebunden sind, einen 5-oder 6-gliedrigen heterocyclischen Ring bilden, der als Ringglied zusätzlich die Atome oder Gruppen -O-, -S-, -SO-, $-SO_2-$,

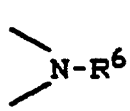 oder 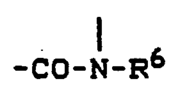

enthalten kann und der gegebenenfalls an den Kohlenstoffatomen ein-bis dreifach, gleich oder verschieden, durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminometyl und Ethylaminomethyl substituiert sein kann, wobei

$R^6$ für Wasserstoff, eine verzweigte oder unverzweigte Alkyl-, Alkenyl-oder Alkinylgruppe mit 1 bis 6 Kohlenstoffatomen, die gegebenenfalls durch ein oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen

gegebenenfalls durch Hydroxy, Methoxy, Chlor oder Fluor ein-oder zweifach substituierten Phenacylrest, einen Oxoalkylrest mit bis zu 6 Kohlenstoffatomen steht, ferner einen Rest $COR^7$ oder $SO_2R^8$ bedeutet, wobei $R^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstofatomen, Amino, Alkylamino oder Dialkylamino mit 1 bis 5 Kohlenstoffatomen im Alkylteil und
$R^8$ geradkettiges oder verzweigtes Alkyl mit 1 bis 3 Kohlenstoffatomen darstellt, oder die Gruppe

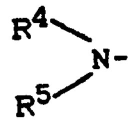

auch für ein Ringsystem der Struktur

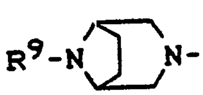
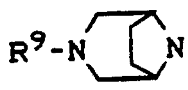
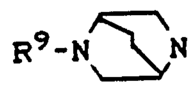
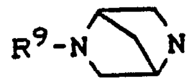
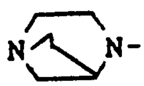
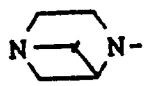

stehen kann, das gegebenenfalls an den Ring-Kohlenstoffen durch Methyl substituiert sein kann, worin
$R^9$ für Wasserstoff, Methyl, Ethyl, Hydroxyethyl, Benzyl oder p-Aminobenzyl stehen kann und deren pharmazeutisch verwendbaren Hydraten oder Salzen, vorzugsweise Alkali-, Erdalkali-, Silber-und Guanidiniumsalzen, sowie ihren Estern zur Herstellung von Arzneimitteln.

Europäisches Patentamt

# EUROPÄISCHER RECHERCHENBERICHT

Nummer der Anmeldung

EP 88 10 0503

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 167 763 (BAYER AG)<br>* Ansprüche 1, 8-14 *<br>--- | 1-3,5-7 | C 07 D 215/56<br>A 61 K 31/47 |
| Y | PATENT ABSTRACTS OF JAPAN, Band 4, Nr. 155 (C-29) [637], 29. Oktober 1980; & JP - A - 55 100 364 (DAINIPPON SEIYAKU K.K.) 31.07.1980<br>----- | 1-3,5-7 | |

| RECHERCHIERTE SACHGEBIETE (Int. Cl.4) |
|---|
| C 07 D 215/00 |

Der vorliegende Recherchenbericht wurde für alle Patentansprüche erstellt

| Recherchenort | Abschlußdatum der Recherche | Prüfer |
|---|---|---|
| BERLIN | 23-03-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)